# EUROPÄISCHE PATENTANMELDUNG

(11) **EP 1 658 873 A1**
(43) Veröffentlichungstag der Anmeldung: **24.05.2006**
(21) Anmeldenummer: 04027683.4
(22) Anmeldetag: 22.11.2004
(51) Int. Cl.: A61M 16/06

(54) **Maske für eine Atemvorrichtung zur Behandlung der Schlaf-Apnoe**

(71) Anmelder: Schumacher, Gerhard, 32257 Bünde (DE)
(72) Erfinder: Schumacher, Gerhard, 32257 Bünde (DE)
(74) Vertreter: Schmitz, Hans-Werner

(57) **Zusammenfassung**

Die Erfindung betrifft eine Maske (1) für eine Atemvorrichtung zur Behandlung von Schlaf-Apnoe, mit einem Maskenkörper (2) und mit einer Befestigungseinrichtung (3), die eine Kopfbandanordnung (4) aufweist, die mit Anschlussteilen (5, 6, 7) zur zumindest temporären Fixierung am Maskenkörper (2) versehen ist, wobei die Befestigungseinrichtung (3) ferner eine Adapterplatte (8) aufweist, der zumindest temporär am Maskenkörper (2) festlegbar ist und an der die Anschlussteile (5, 6, 7) der Kopfbandanordnung (4) fixierbar sind.

## Beschreibung

Die Erfindung betrifft eine Maske für eine Atemvorrichtung zur Behandlung der Schlaf-Apnoe gemäß dem Oberbegriff des Anspruchs 1.

Eine derartige Maske ist aus der WO 82/03548 bekannt. Der Maskenkörper dieser Maske wird durch eine Kopfband-Anordnung auf der Nase der zu behandelnden Person fixiert, die bei der gattungsgemäßen Maske an Luftzuführleitungen zum Nasen- bzw.
Luftdruckraum des Maskenkörpers führen. Diese Befestigungsmethode kann jedoch vor allem bei Bewegungen, die der Träger in der Nacht ausführt, eine sichere Festlegung der Nasenmaske nicht ausreichend gewährleisten.

Daher gibt es Maskenkonstruktionen, bei denen die Bandanordnung direkt am Maskenkörper angreift. Hierbei muss der Maskenkörper mit geeigneten Anschlusseinrichtungen, wie beispielsweise Laschen, Bügeln, Stirnstützen oder Ähnlichem versehen sein, an denen die Bandanordnung angreifen kann (siehe beispielsweise WO 99/64096).

Der Nachteil dieser Konstruktion ist jedoch darin zu sehen, dass häufig der Tragekomfort eingeschränkt ist, da relativ große Kräfte auf den Maskenkörper und damit auf das Gesicht des Trägers aufgebracht werden. Ferner ist es bei derartigen Anordnungen, die als Zweipunkt-, Dreipunkt- oder auch Vierpunkt-Anordnungen ausgebildet sein können, nachteilig, dass eine Zentrierung des Maskenkörpers auf der Nase bzw. dem Gesicht des Trägers zur Einnahme einer optimalen Maskenposition schwierig bzw. teilweise sogar unmöglich ist.

Es ist daher Aufgabe der vorliegenden Erfindung, eine Maske der im Oberbegriff des Anspruches 1 angegebenen Art zu schaffen, deren Befestigungseinrichtung unabhängig von der Form des Maskenkörpers eine optimale Zentrierung der Maske auf dem Gesicht des Trägers bei hohem Tragekomfort ermöglicht.

Die Lösung dieser Aufgabe erfolgt durch die Merkmale des Anspruchs 1.

Durch das Vorsehen eines Adapters, vorzugsweise in Form einer Adapterplatte bzw. eines Adapterrings, wird eine Fixierung mit wenig Kraft direkt im Zentrum der Maske ermöglicht. Eine Konzentration der Zugkräfte von drei Haltebändern im oberen Bereich der Atemmaske werden zumindest ungefähr auf den Mittelpunkt der Maske konzentriert, und somit problemlos und vor allem gleichmäßig und sanft positioniert und in der richtigen Position gehalten.

Es ist vom Prinzip her möglich, den Adapter nur auf die Maske aufzustecken, beispielsweise mit Hilfe einer Ausnehmung der Adapterplatte, die über einen Luftzuführschlauch oder einen Luftzuführanschlussstutzen gesteckt werden kann.

Alternativ ist es möglich, den Adapter auf den Maskenkörper dauerhaft zu fixieren, beispielsweise durch eine Verschraubung, oder der Form und Wirkung entsprechend, direkt in den Maskenkörper zu integrieren.

Mit dem Adapter wird es möglich, die Krafteinleitung zumindest ungefähr am höchsten Punkt des Maskenkörpers, gesehen aus der Sicht des Maskenträgers, anzubringen. Dies verbessert weiter die Krafteinleitung und den Tragekomfort.

Die Unteransprüche 2 bis 9 haben vorteilhafte Weiterbildungen der Erfindung zum Inhalt.

In Anspruch 10 ist ein Adapter als selbständig handelbares Objekt definiert, da der Adapter mit einer Vielzahl unterschiedlicher Masken kombinierbar ist, ohne von den Prinzipien vorliegender Erfindung abweichen zu müssen.

Weitere Einzelheiten, Vorteile und Merkmale der vorliegenden Erfindung ergeben sich aus nachfolgender Beschreibung von Ausführungsbeispielen anhand der beigefügten Zeichnung. Darin zeigt:
- Fig. 1: eine mit einer erfindungsgemäßen Adapterplatte fixierte Atemmaske an einem Modellkopf;
- Fig. 2: eine perspektivische Darstellung der Maske gemäß Fig. 1 im unmontierten Zustand;
- Fig. 3: eine Draufsicht auf einen Adapter der erfindungsgemäßen Maske;
- Fig. 4: eine der Fig. 3 entsprechende Darstellung auf einer alternativen Ausführungsform einer Adapterplatte;
- Fig. 5: eine Darstellung der Adapterplatte gemäß Fig. 3 mit einer geometrischen Erläuterung der Lage eines Mittelpunktes einer Ausnehmung der Adapterplatte; und
- Fig. 6: eine Draufsicht auf eine zweite Ausführungsform eines erfindungsgemäßen Adapters in Form eines Adapterrings.

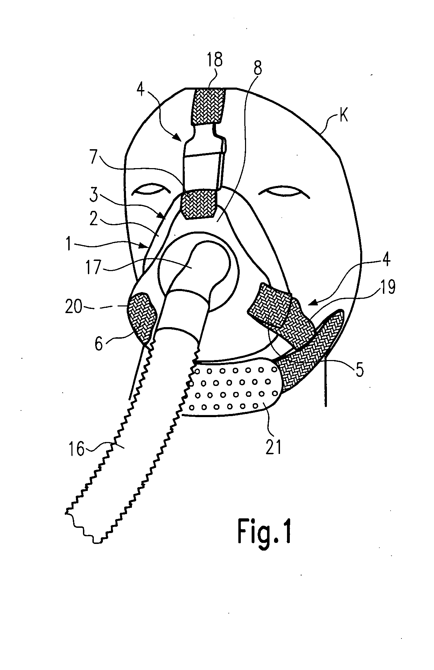

In Fig. 1 ist eine erfindungsgemäße Adaption ab einer Maske 1 dargestellt, die zur Illustrierung ihrer Fixierungsmöglichkeit an einem Modellkopf K angebracht ist.

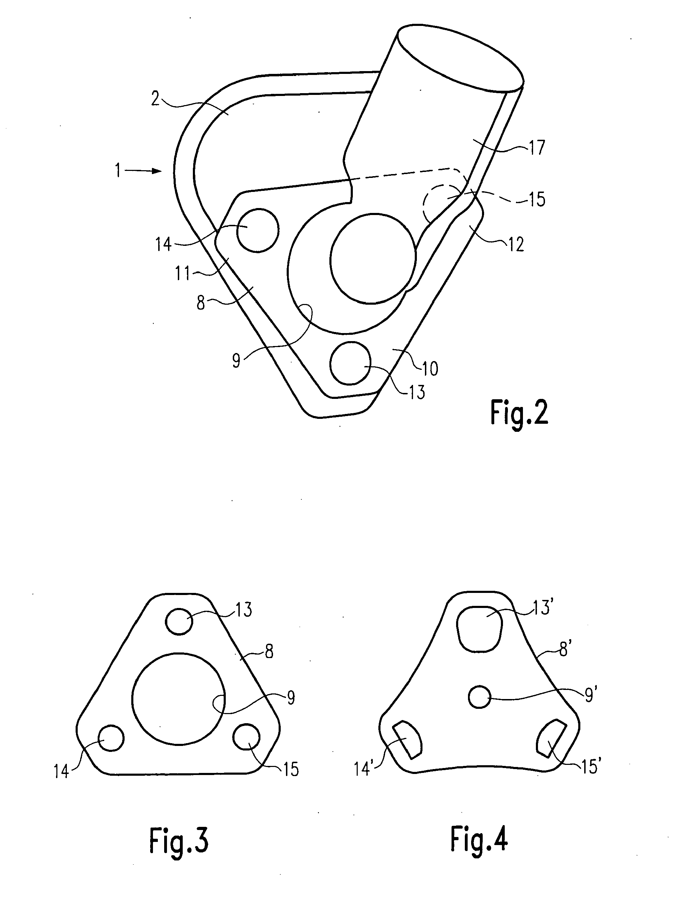

Die Maske 1 selber, die in Fig. 2 im unmontierten Zustand ebenfalls dargestellt ist, weist einen Maskenkörper 2 auf. Der Maskenkörper 2 kann unterschiedlichste Formen haben, weist in jedem Falle jedoch einen Innen- bzw. Druckraum auf, in dem zur Behandlung der Schlaf-Apnoe ein Überdruck erzeugt wird. Hierzu ist die Maske 1 mit einer in den Figuren nicht dargestellten C PAP- oder bi Level PAP-Geräten verbunden, die über einen Luftschlauch 16 und einen Schlauchanschlussstutzen 17 mit dem Innenraum des Maskenkörpers 2 verbunden ist. Ferner weist die Maske 1 eine erfindungsgemäße Befestigungseinrichtung 3 auf, die mit einer Kopfbandanordnung 4 versehen ist. Die Kopfband-anordnung 4 weist zur Herstellung einer Dreipunkt-Befestigung drei Bänder 18, 19, 20 auf, die in üblicher Art und Weise ausgebildet sein können.

Die Bänder werden über Anschlussteile 5, 6 bzw. 7 an einer Adapterplatte 8 befestigt, die zumindest temporär am Maskenkörper 2 festgelegt wird. Der Adapterplatte 8, die zusätzlich separat in Fig. 3 dargestellt ist, weist eine im Wesentlichen dreieckige Form auf. Wie die Figuren verdeutlichen, ist die Adapterplatte 8 aufgrund ihrer Dreiecksform mit drei Eckbereichen 10, 11 und 12 versehen, die jeweils abgeplattet sind. Ferner weist die Adapterplatte 8 bei der in den Figuren 1 bis 3 dargestellten Ausführungsform eine mittige Ausnehmung 9 auf, die zur Festlegung am Maskenkörper 2 über den Anschlussstutzen 17 gesteckt werden kann, so wie sich dies im Einzelnen aus Fig. 2 ergibt. Die Größe der Ausnehmung 9 wird hierbei natürlich jeweils an die Größe des Anschlussstutzens 17 angepasst.

Dadurch ist es möglich, die Adapterplatte 8 an der höchsten Stelle des Maskenkörpers 2 anzuordnen und somit eine perfekte Zentrierung und Krafteinleitung möglich zu machen. Ferner weist die Adapterplatte 8 drei in den Eckbereichen 10, 11 und 12 angeordnete Anschlussausnehmungen 13, 14 bzw. 15 auf, die mit den Anschlussteilen 5, 6 bzw. 7 zusammenwirken, wie sich dies aus Fig. 1 ergibt.

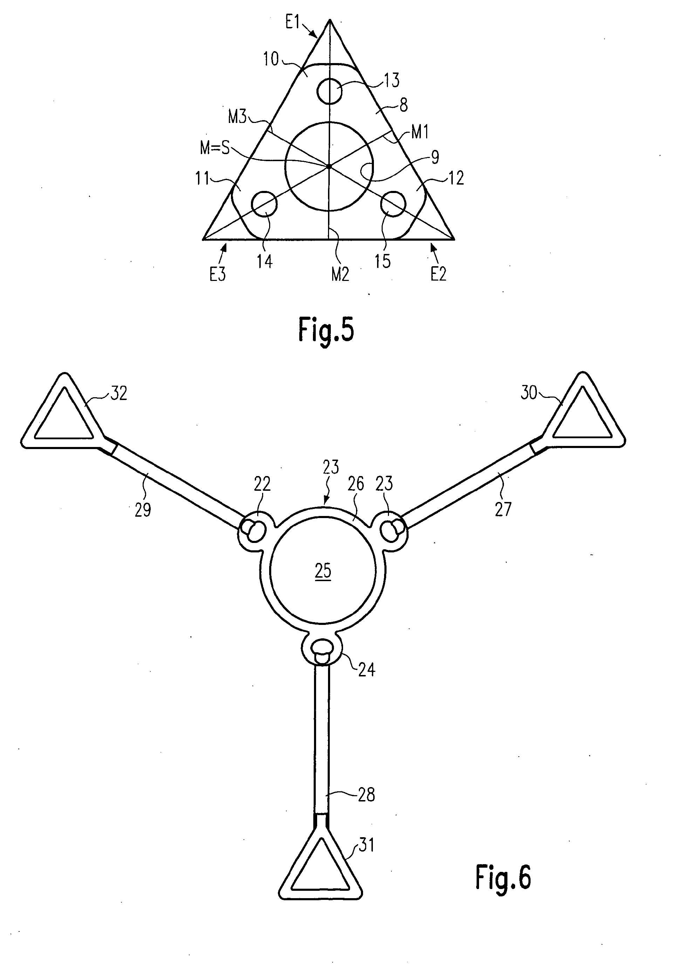

Um die zuvor erläuterte Krafteinleitung und Zentrierung der Adapterplatte und damit der Maske 1 selber auf dem Bereich der Nase des Trägers zu erreichen, ist in Fig. 5 eine geometrische Erläuterung anhand der Adapterplatte 8 der Fig. 3 wiedergegeben, aus der sich ergibt, dass der Schwerpunkt S der Adapterplatte mit dem Mittelpunkt M der Ausnehmung 9 zusammenfällt. Hierzu ist in Fig. 5 in den Eckbereichen 10, 11 und 12, die, wie zuvor erläutert, abgeplattet sind, jeweils ein zugeordneter geometrischer Eckbereich E1, E2 bzw. E3 ergänzt. Ferner wurden die jeweiligen Mittelpunkte M1, M2 und M3 der Dreiecksseiten eingezeichnet und diese mit den gegenüberliegenden Eckpunkten verbunden, wie sich dies im Einzelnen aus der geometrischen Darstellung der Fig. 5 ergibt. Dies ergibt den Schwerpunkt S, der, wie zuvor erläutert, mit dem Mittelpunkt M der Ausnehmung 9 zusammenfällt.

Die Adapterplatte 8, die in den Fig. 1, 2 und 3 dargestellt ist, wird lediglich auf den Anschlussstutzen 17 gesteckt, ohne dass weitere Befestigungen erforderlich wären.

In Fig. 4 ist eine alternative Ausführungsform einer Adapterplatte 8' dargestellt, die ebenfalls eine dreieckige Form mit abgeplatteten Eckbereichen und drei Anschlussausnehmungen 13' bis 15' aufweist.

Die mittige Ausnehmung 9', deren Mittelpunkt wiederum mit dem Schwerpunkt S zusammenfällt, ist jedoch deutlich kleiner als die Ausnehmung 9 der Ausführungsform gemäß Fig. 3.

Diese Ausnehmung 9' kann als Durchtrittsausnehmung beispielsweise für eine Schraube oder einen Aufnahmestift dienen, mit Hilfe derer die Adapterplatte 8' an einer Maske bzw. Maskenkörper 2 dauerhaft fixiert werden kann.

In Fig. 6 ist eine zweite Ausführungsform des erfindungsgemäßen Adapters dargestellt, bei der es sich um einen Adapterring 21 handelt. Der Adapterring 21 weist an seinem Umfang drei in einem Winkel von 120° beabstandete Befestigungsaugen 22, 23 und 24 auf, die vorzugsweise einstückig mit dem Ringkörper 26 verbunden sind. Der Ringkörper 26 weist eine mittige Ausnehmung 25 auf, die beispielsweise wiederum über den Anschlussstutzen 17 gesteckt werden kann.

An den Befestigungsaugen 22, 23 und 24 sind Silikonbänder 27, 28 bzw. 29 befestigt, an denen wiederum Befestigungslaschen 30, 31 und 32 zum Anschluss an die Kopfbandanordnung vorgesehen sind. Wie eingangs erläutert, kann auch mit diesem Adapter eine Fixierung mit wenig Kraft direkt im Zentrum der Maske erfolgen.

Insgesamt ist somit zu sagen, dass die Adapterplatte 8 an die jeweiligen Einbauverhältnisse unterschiedlichster Maskenkörper hinsichtlich Form und Größe anpassbar ist.

Als Materialien kommen Metalle, insbesondere Leichtmetalle wie Aluminium oder jedwede Art von geeigneten Kunststoffmaterialien für den Adapter in Frage.

### Bezugszeichenliste

- 1: Maske
- 2: Maskenkörper
- 3: Befestigungseinrichtung
- 4: Kopfbandanordnung
- 5, 6, 7: Anschlussteile
- 8, 8': Adapterplatte
- 9: Halteausnehmung
- 10, 11, 12: Abgeplattete Eckbereiche
- 13, 13', 14, 14', 15, 15': Anschlussausnehmungen
- 16: Luftrohr
- 17: Anschlussstutzen
- 18, 19, 20: Bänder
- 21: Kinnstütze
- 22, 23, 24: Befestigungsaugen
- 25: Mittige Ausnehmung
- 26: Ringkörper
- 27, 28, 29: Silikonbänder
- 30, 31, 32: Befestigungslaschen
- 33: Adapterring
- K: Kopfmodell

## Patentansprüche

1. Maske (1) für eine Atemvorrichtung zur Behandlung von Schlaf-Apnoe,
- mit einem Maskenkörper (2); und
- mit einer Befestigungseinrichtung (3), die eine Kopfbandanordnung (4) aufweist, die mit Anschlussteilen (5, 6, 7) zur zumindest temporären Fixierung am Maskenkörper (2) versehen ist,
**dadurch gekennzeichnet,**
- **dass** die Befestigungseinrichtung (3) ferner einen Adapter (8; 8'; 21) aufweist, der zumindest temporär am Maskenkörper (2) festlegbar ist und an der die Anschlussteile (5, 6, 7) der Kopfbandanordnung (4) fixierbar sind.

2. Maske nach Anspruch 1, **dadurch gekennzeichnet, dass** der Adapter als Adapterplatte (8; 8') ausgebildet ist, die zumindest ungefähr am höchsten Punkt des Maskenkörpers (2) platzierbar ist.

3. Maske nach Anspruch 1 oder 2, **dadurch gekennzeichnet, dass** die Adapterplatte (8; 8') eine im Wesentlichen dreieckige Form aufweist.

4. Maske nach Anspruch 3, **dadurch gekennzeichnet, dass** die Adapterplatte (8; 8') eine Halteausnehmung (9; 9') aufweist.

5. Maske nach Anspruch 4, **dadurch gekennzeichnet, dass** die Halteausnehmung (9) einen Mittelpunkt (M) aufweist, der im Schwerpunkt (S) der Adapterplatte (8; 8') liegt.

6. Maske nach einem der Ansprüche 3 bis 5, **dadurch gekennzeichnet, dass** die Adapterplatte (8; 8') in ihren drei Eckbereichen (10, 11, 12) Anschlussausnehmungen (13, 14, 15; 13', 14', 15') für die Anschlussteile (5, 6, 7) der Kopfbandanordnung (4) aufweist.

7. Maske nach einem der Ansprüche 1 bis 6, **dadurch gekennzeichnet, dass** die Adapterplatte (8; 8') aus Metall, insbesondere Leichtmetall besteht.

8. Maske nach einem der Ansprüche 1 bis 6, **dadurch gekennzeichnet, dass** die Adapterplatte (8; 8') aus Kunststoff besteht.

9. Maske nach einem der Ansprüche 3 bis 8, **dadurch gekennzeichnet, dass** die Adapterplatte (8; 8') abgeplattete Endbereiche (10, 11, 12) aufweist.

10. Maske (1) nach Anspruch 1, **dadurch gekennzeichnet, dass** der Adapter als Adapterring (33) ausgebildet ist, der mit Befestigungsaugen (22 bis 24) versehen ist, die am Umfang des Ringkörpers (26) in 120°-Abständen angeordnet sind.

11. Adapter nach Anspruch 10, **dadurch gekennzeichnet, dass** die Befestigungsaugen (22 bis 24) mit Silikonbändern (27 bis 29) oder anderen hochflexiblen Bändern versehen sind, an denen Befestigungslaschen (30 bis 32) vorgesehen sind.

12. Adapter (8; 8'; 21) für eine Maske (1) gemäß dem Oberbegriff des Anspruches 1, **gekennzeichnet durch** wenigstens eines der Merkmale 1 bis 11.
